(19) Europäisches Patentamt
European Patent Office
Office européen des brevets

(11) Veröffentlichungsnummer: **0 087 737**
**B1**

(12) # EUROPÄISCHE PATENTSCHRIFT

(45) Veröffentlichungstag der Patentschrift: **09.01.85**

(21) Anmeldenummer: **83101716.5**

(22) Anmeldetag: **23.02.83**

(51) Int. Cl.⁴: **C 07 C 43/17**, C 07 C 59/135, C 08 F 16/26, C 08 F216/14, C 07 C 41/24, C 07 C 51/62

(54) **Perfluorierte Vinylether mit einem sekundären Wasserstoffatom, Polymere daraus, sowie Verfahren zur Herstellung der Monomeren.**

(30) Priorität: **27.02.82 DE 3207143**

(43) Veröffentlichungstag der Anmeldung: **07.09.83 Patentblatt 83/36**

(45) Bekanntmachung des Hinweises auf die Patenterteilung: **09.01.85 Patentblatt 85/02**

(84) Benannte Vertragsstaaten: **BE CH DE FR GB IT LI NL**

(56) Entgegenhaltungen:
**DE-A- 1 793 181**
**DE-A- 1 806 097**
**DE-A- 2 639 109**

(73) Patentinhaber: **HOECHST AKTIENGESELLSCHAFT**
**Postfach 80 03 20**
**D-6230 Frankfurt am Main 80 (DE)**

(72) Erfinder: **Siegemund, Günter, Dr.**
**Frankfurter Strasse 21**
**D-6238 Hofheim am Taunus (DE)**
Erfinder: **Schwertfeger, Werner, Dr.**
**Erlenstrasse 8**
**D-6306 Langgöns (DE)**

**EP 0 087 737 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Perfluorierte Vinylether sind Verbindungen mit der an einem perfluorierten organischen Rest gebundenen —O—CF=$CF_2$ Gruppe. Sie werden meist durch Homo- oder Copolymerisation (vorzugsweise mit ebenfalls perfluorierten ungesättigten Monomeren) zu Polymerisaten mit wertvollen anwendungstechnischen Eigenschaften verarbeitet.

Unter den z. B. nach den Polymerisationsverfahren gemäß DE-AS 1 806 097 und DE-OS 26 39 109 weiterverarbeiteten perfluorierten Vinylethern sind u. a. auch solche, welche noch ein primäres Wasserstoffatom besitzen ; diese perfluorierten Vinylether sind :

$$CF_2 = CF-O-(CF_2)_n-CF_2H \quad \text{und}$$

$$CF_2=CF-O-CF_2-\underset{}{\overset{CF_3}{\overset{|}{C}}}F-O-(CF_2)_n-CF_2H$$

(n = 0-10)

Wenn die perfluorierten Vinylether noch eine Estergruppe im Molekül enthalten, entstehen nach der Polymerisation Polymerisate mit Estergruppen, welche auf bekannte Weise verseifbar sind. Die resultierenden Carboxylgruppen enthaltenden Fluorkohlenstoffpolymerisate besitzen Kationenaustauschereigenschaften und werden daher hauptsächlich als Kationenaustauschermembranen in Elektrolysezeller insbesondere bei der Chloralkalielektrolyse verwendet. Ein beispielhafter Herstellungsweg für solche perfluorierten Kationenaustauscher ist beschrieben in dem Artikel von Maomi Seko « Ion-Exchange Membrane For the Chlor-Alkali-Process » (vorgelegt auf der 159. Sitzung der Elektrochemical Society Minneapolis, Minnesota, vom 13 Mai 1981) ; dieser Herstellungsweg wird nachstehend wie folgt wiedergegeben :

(Siehe Schema Seite 3 f.)

Schema

1) Synthese von $CF_2=CFO(CF_2-CF(CF_3)O)_m(CF_2)_nCOOR$

m = 0 oder 1, n = 1-4

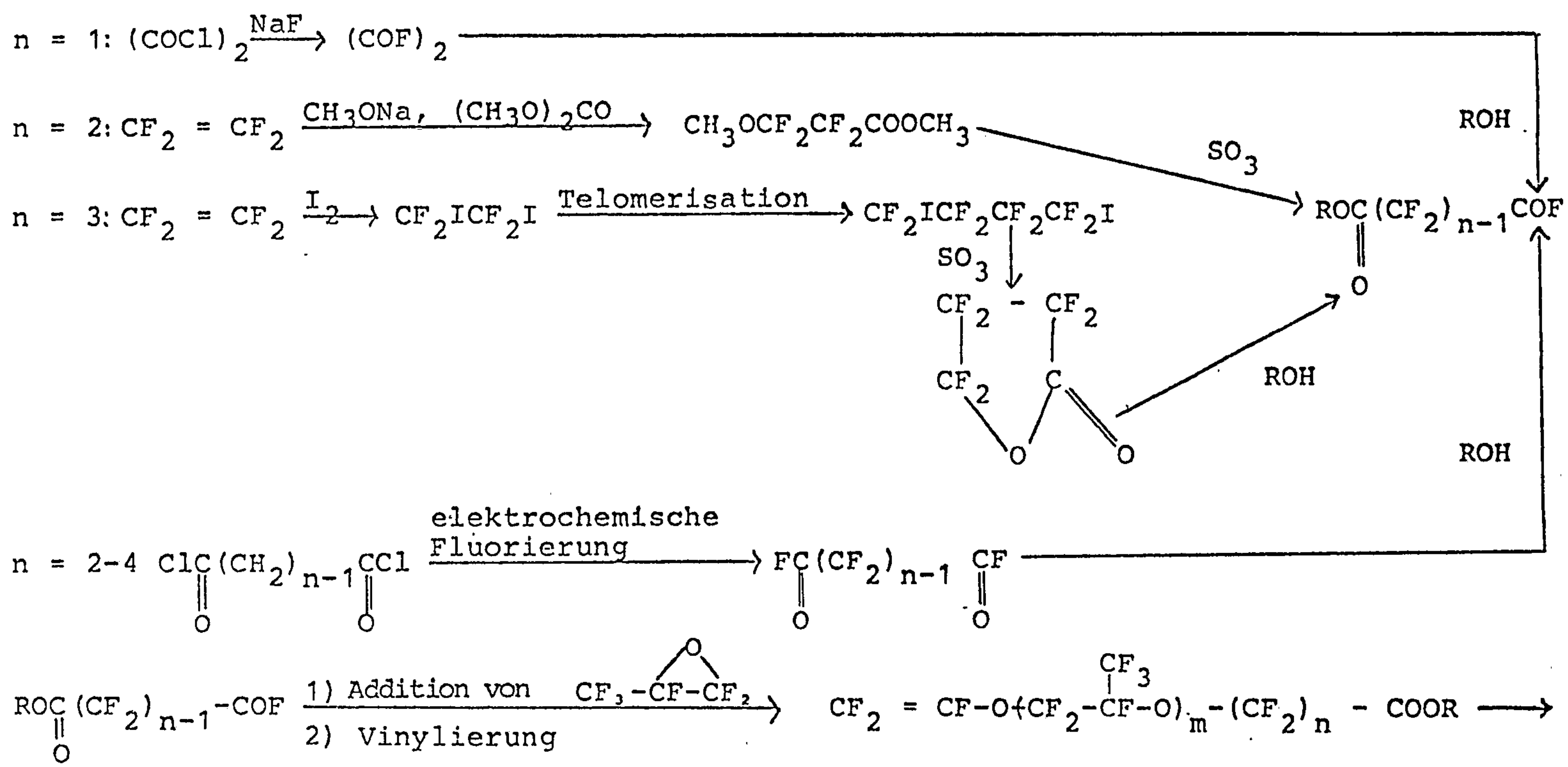


2) Weiterverarbeitung des Vinylmonomeren

Copolymerisation mit $CF_2 = CF_2$ → Copolymer mit COOR-Gruppen → (Extrusion und Verseifung) COOH-Gruppen enthaltende Kationenaustauscher

Der für die Herstellung Carboxylgruppen enthaltender perfluorierter Kationenaustauscher typische Syntheseweg zeigt, daß die Carbonsäuregruppe in Form der reaktiven Estergruppe bereits in einer frühen Stufe des Synthesewegs — jedenfalls erheblich vor der Polymerisation — eingeführt werden muß. Bei allen auf die Einführung der Carbonestergruppe folgenden Reaktionsstufen ist daher die Reaktivität dieser Gruppe zu berücksichtigen und gegebenenfalls ihr Erhalt durch Anwendung besondere Maßnahmen sicherzustellen.

Es bestand daher die Aufgabe, einen Weg zu finden, bei dem die Ester- bzw. Carboxylgruppe erst am Ende der gesamten Synthese der entsprechenden perfluorierten Kationenaustauscher eingeführt wird.

Die Lösung dieser Aufgabe ist zwar im Prinzip über die bekannten perfluorierten Vinylether mit einem primären Wasserstoffatom im Molekül möglich, jedoch nicht in einem den erheblichen Bedarf an günstigeren und billigeren Verfahren zur Herstellung von perfluorierten Kationenaustauschern befriedigenden Maß.

Ein wesentlicher weiterer Beitrag zur Lösung der gestellten Aufgabe wurde daher erfindungsgemäß durch die Bereitstellung einer Reihe neuer perfluorierter Vinylether mit einem sekundären Wasserstoffatom geleistet ; die neuen Verbindungen besitzen die nachstehende Formel I :

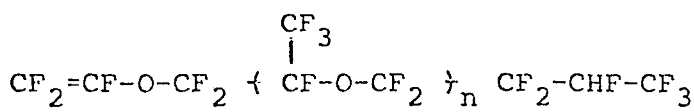

(I)

worin n = 0-5, vorzugsweise 0-3, insbesondere 0-2.

Die Verbindungen lassen sich nach bekannten Verfahren — wie z. B. beschrieben in den eingangs erwähnten Druckschriften DE-AS 1 806 097 und DE-OS 2 639 109 — homo- und copolymerisieren (letzteres z. B. mir $CF_2{=}CF_2$). Die — ebenfalls neuen — Polymerisate bestehen dann aus Makromolekülen mit den Seitenkettenendgruppen $—CHF—CF_3$, welche sich nach dem Verfahren einer gleichzeitig eingereichten Patentanmeldung der gleichen Anmelderin funktionalisieren und in Carboxylgruppen umwandeln lassen ; dies geschieht durch Umsetzung der Polymerisate (mit den Seitenketten-Endgruppen $—CHF—CF_3$) mit Peroxodisulfuryldifluorid $FSO_2O—OSO_2F$ zu den entsprechenden Fluorsulfatoderivaten mit den Seitenkettenendgruppen

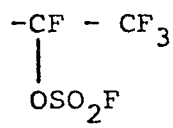

und Zersetzung derselben in Gegenwart katalytischer Mengen von Alkalifluoriden und/oder von aprotischen Stickstoffbasen zu den entsprechenden Ketonen mit den Seitenkettenendgruppen

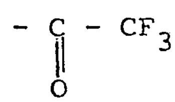

(in denen die Ketogruppe in Gegenwart von Wasser oder Alkoholen auch als Hydrat resp. Halbketal vorliegen kann) und Hydrolyse der Ketone zu den Produkten mit der Carboxylgruppe (vorzugsweise in stark basischem Medium) :

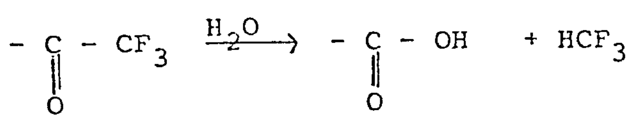


Die neuen Verbindungen der Formel I sowie die Polymerisate daraus ermöglichen so einen vorteilhaften und fortschrittlichen Zugang zu Carboxylgruppen enthaltenden perfluorierten Kationenaustauscher-Polymerisaten. Vorteilhaft und fortschrittlich ist dieser Zugang insbesondere deswegen, weil die Einführung der Carboxylgruppen — im Gegensatz zum einschlägigen Stand der Technik — erst am Ende des gesamten Synthesewegs erfolgt.

Die neuen Verbindungen der Formel I werden erfindungsgemäß hergestellt durch

a) Umsetzung von 3-H-Perfluorbutansäurefluorid (II)

$$FOC—CF_2—CHF—CF_3 \qquad (II)$$

mit Hexafluorpropenepoxid (III)

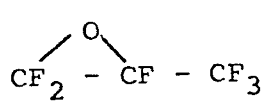

(III)

in Gegenwart mindestens eines ionogenen Fluorids als Katalysator sowie eines inerten aprotisch-polaren Lösungsmittels bei Temperaturen zwischen − 30 und + 100 °C, vorzugsweise zwischen 0 und + 50 °C, zu dem Säurefluorid der Formel IV

$$FOC\left(\underset{\displaystyle CF}{\overset{\displaystyle CF_3}{|}}-O-CF_2\right)_{n+1}CF_2-CHF-CF_3 \qquad (IV)$$

worin n die gleiche Bedeutung wie in Formel I besitzt,

b) Pyrolyse des Säurefluorids IV als solchem oder nach Umwandlung in das entsprechende Alkalicarboxylat bei Temperaturen zwischen 100 und 600 °C und

c) Isolierung des bei der Pyrolyse gebildeten Vinylethers der Formel I.

Die Stufen a) und b) des Verfahrens lassen sich formelmäßig wie folgt wiedergeben (schematisch) :

a) 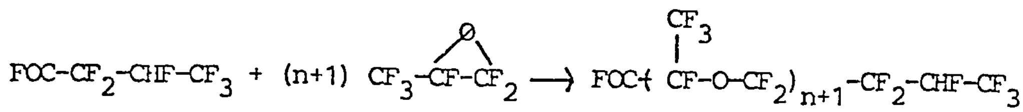

(II) (III) (IV)

b) 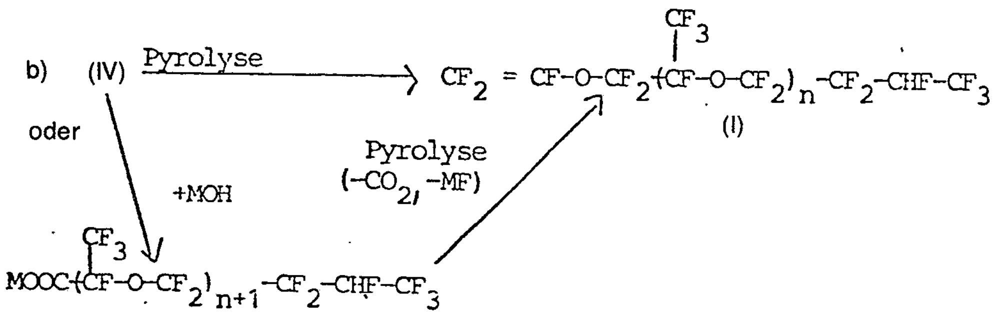


(M = Alkalimetallion)

Die Ausgangsverbindung für das Verfahren — das 3-H-Perfluorbutansäurefluorid (II) — ist eine bekannte Verbindung, welche auf folgendem Syntheseweg zugänglich ist [J. Amer. Chem. Soc. *77* (1955), S. 9100 ff und Umsetzung des Säurechlorids $ClOC—CF_2—CHF—CF_3$ ins Säurefluorid auf bekannte Weise] :

$$CF_2=CF—CF_3 \xrightarrow{+\,CH_3OH\ (Radikalstarten)} HOH_2C—CF_2—CHF—CF_3 \xrightarrow{KMnO_4} KOOC—CF_2—$$

$$CHF—CF_3 \xrightarrow{+\,C_6H_5CCl_3} ClOC—CF_2—CHF—CF_3 \xrightarrow{+\,KF/CH_3CN} FOC—CF_2—CHF—CF_3 \qquad (II)$$

Die Durchführung der einzelnen Stufen des erfindungsgemäßen Verfahrens zur Herstellung der Verbindungen I kann im Prinzip nach den für solche Reaktionen üblichen Methoden erfolgen, wie z. B. beschrieben in US-PS 3 114 778 für das dortige Verfahren zur Herstellung von Perfluorvinylethern aus Perfluoralkansäurefluoriden und Hexafluorpropenepoxid sowie Pyrolyse der gebildeten Reaktionsprodukte als solchen oder in Form der entsprechenden Alkalicarboxylate.

Demnach sind in Stufe a) des erfindungsgemäßen Verfahrens als Katalysatoren im Prinzip alle möglichen ionogenen Fluoride einsetzbar ; vorzugsweise werden jedoch die Alkalifluoride, quartären Ammoniumfluoride und Silberfluorid, insbesondere die Alkalifluoride (und hierunter wieder hauptsächlich Kalium- und Cäsiumfluorid) eingesetzt. Die einzelnen Katalysatorverbindungen können sowohl allein als auch in Mischung verwendet werden.

Die Katalysatormenge bewegt sich im allgemeinen zwischen etwa 0,01 und etwa 5, vorzugsweise zwischen etwa 0,01 und etwa 1 Gew.-%, bezogen auf die Gesamtmenge des eingesetzten Hexafluorpropenepoxids.

Bevorzugte inerte aprotisch-polare Lösungsmittel sind Nitrile (Acetonitril, Benzonitril), Ether (insbesondere Polyalkylether wie Ethylenglykoldimethylether, Diethylenglykoldimethylether und Tetraethylenglykoldimethylether) ; auch Lösungsmittel wie Dimethylsulfoxid und N-Methylpyrolidon sind verwendbar.

Die Reaktionstemperatur kann in relativ weiten Grenzen schwanken ; sie liegt zwischen − 30 und 100 °C, insbesondere zwischen 0 und 50 °C.

Als Reaktionsdruck ist sowohl Normal- als auch Überdruck oder Vakuum möglich ; bevorzugt ist leichter Überdruck oder Normaldruck.

Eine bevorzugte Durchführungsweise der Reaktionsstufe a) besteht darin, daß man das 3-H-Perfluorbutansäurefluorid II, den Katalysator und ein inertes aprotischpolares Lösungsmittel in einem Reaktionsgefäß vorlegt und Hexafluorpropenepoxid III unter Rühren einleitet. Nach beendeter Reaktion wird der Ansatz destillativ aufgearbeitet :

Durchführung von Reaktionsstufe b) : Wenn das in Stufe a) erhaltene Säurefluorid IV als solches direkt pyrolysiert wird, wird eine Temperatur von etwa 300 bis 600 °C angewandt. In Gegenwart von Katalysatoren wie etwa von Natriumsulfat kann die Temperatur auch etwas niedriger liegen.

Die Anwendung einer niedrigeren Temperatur ist auch möglich, wenn das Säurefluorid IV erst in das entsprechende Alkalicarboxylat (z. B. mittels KOH) überführt wird. Das Alkalicarboxylat wird im allgemeinen bei etwa 170 bis 250 °C pyrolysiert, in Gegenwart von inerten Verdünnungsmittels wie z. B. von Paraffinöl bei noch niedrigerer Temperatur (bis herab zu etwa 100 °C).

Als Reaktionsdruck für die Pyrolyse ist Normaldruck oder Vakuum bevorzugt.

Eine bevorzugte Ausführungsform der Reaktionsstufe b) besteht darin, daß man das Säurefluorid IV durch Zutropfen von wäßrigem Alkali verseift, das entstandene Alkalisalz trocknet und bei erhöhter Temperatur im Vakuum pyrolysiert.

Der bei der Pyrolyse gebildete Vinylether I wird dann zweckmäßig in Reaktionsstufe c) durch fraktionierte Destillation isoliert und gereinigt.

Die Ausbeuten des Verfahrens liegen normalerweise zwischen etwa 60 und 70 % d. Th.

Die Erfindung wird nun durch das nachfolgende Beispiel näher erläutert.

## Beispiel

a) Reaktion von 3-H-Perfluorbutansäurefluorid mit Hexafluorpropenepoxid

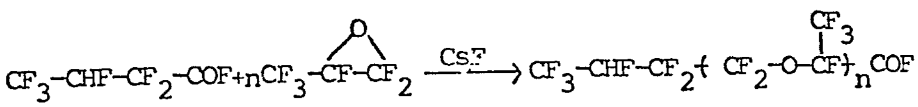


In einem Glasautoklaven, der mit einem Flügelrührer und einem Kühlmantel ausgestattet ist, legt man 30 g (0,2 Mol) CsF, 100 ml Tetraglyme und 740 g (3,73 Mol) $CF_3$—CHF—$CF_2$—COF vor. Unter gutem Rühren drückt man Hexafluorpropenepoxid (HFPO) auf. Die Aufnahme des Gases erfolgt sofort unter exothermer Reaktion. Die Innentemperatur wird durch Kühlen, bei etwa 30 °C gehalten. Insgesamt werden 1 050 g (6,3 Mol) HFPO eingegast. Dafür benötigt man 1,5 Stunden. Der Ansatz rührt ca. eine Stunde bei RT nach und wird anschließend über eine Füllkörperkolonne destilliert. Dabei werden die folgenden Verbindungen erhalten :

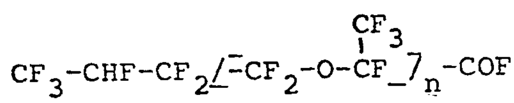

n = 1 : Kp 90-93 °C / 1 007 mbar (755 Torr) ; 655 g = 48 % d. Th.
n = 2 : Kp 82-84 °C / 133 mbar (100 Torr) ; 455 g = 23 % d. Th.

Ein weiterer Ansatz mit 751 g (3,8 Mol) $CF_3$—CHF—$CF_2$—COF, 20 g (0,13 Mol) CsF, 100 ml Tetraglyme und 1 909 g (11,5 Mol) HFPO (das HFPO wird bei Normaldruck eingegast) liefert folgende Fraktionen :

n = 1 : 404 g = 29 %
n = 2 : 774 g = 38 %
n = 3 : 307 g = 12 % Kp 85-88 °C / 28 mbar (21 Torr)

$b_1$) 3-H-Perfluor-n-butylvinylether

$$CF_3—CHF—CF_2—CF_2—O—CF=CF_2$$

399 g (1,1 Mol) 6-H-3-Oxa-perfluor-2-methyl-heptansäurefluorid — d. i. die in Stufe a) hergestellte Verbindung

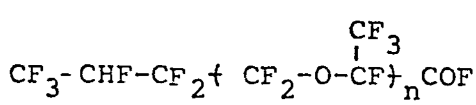

mit n = 1 — werden bei 20-40 °C mit wäßriger Natronlauge umgesetzt, bis zugesetztes Phenolphthalein über 30 Minuten eine schwache Rotfärbung zeigt. Nach dem Eindampfen der Lösung wird das erhaltene Salz im Vakuum bei ca. 100 °C getrocknet. Das pulverisierte Salz suspendiert man in einem Liter Paraffinöl und erhitzt auf ca. 200 bis 250 °C. Unter $CO_2$-Abspaltung entsteht der Vinylether, der zum großen Teil

$c_1$) abdestilliert. Eine kleine Menge Vinylether kondensiert auch in der nachgeschalteten Kältefalle (– 78 °C). Die Destillation ergibt 205 g (63 %) reinen Vinylether mit Kp 76-78 °C/1 013 mbar (760 Torr).

$^1$H-NMR : = 5,05 (dm, CHF, J = 44 Hz)

$^{19}$F-NMR : = – 74,8 (m, 3F, $CF_3$), – 86,8 (dm, 1F, —CHF—$C\underline{F}_2$—, $^2$J = 140 Hz), – 89,3 (dm, 1F, —CHF—$C\underline{F}_2$—, $^2$J = 140 Hz), – 114,3 (dd, 1F, —O—CF=$C\underline{F}_2$ trans, J = 87 und 65 Hz), – 122,4 (ddm, 1F, —O—$C\underline{F}$=$CF_2$, J = 110 und 87 Hz), – 124,2 (dm , 1F, —$CF_2$—O—, $^2$J = 285 Hz), – 131,7 (dm, 1F, —$CF_2$—O—, $^2$J = 285 Hz), – 135,9 (ddm, 1F, —O—CF=$C\underline{F}_2$ cis, J = 110 und 65 Hz), – 213,5 (m, 1F, CHF)

$b_2$) 2-(3-H-Perfluor-n-butoxy)-perfluorpropylvinylether

$$CF_3—CHF—CF_2—CF_2—O—CF(CF_3)—CF_2—O—CF=CF_2$$

765 g (1,44 Mol) 9-H-3,6-Dioxa-perfluor-2,5-dimethyldecansäurefluorid — d. i. die in Stufe a) hergestellte Verbindung

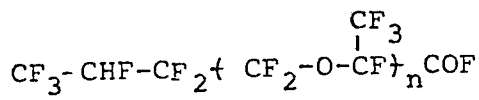 (I)

mit n = 2 — werden, wie in Abschnitt $b_1$ beschrieben, in das Natriumsalz umgewandelt. Das trockene Salz pyrolysiert man bei einem Druck von 7 mbar (5 Torr) und einer Ölbadtemperatur von ca. 230 °C.

$c_2$) Die Reinigung des Rohpyrolysats erfolgt durch Destillation über eine gute Kolonne. Mit Kp 57-58 °C/67 mbar (50 Torr) werden 423 g (63 %) Vinylether erhalten.

$^1$H-NMR : = 5,00 (dm, CHF, J = 44 Hz)

$^{15}$F-NMR : = – 75,8 (m, 3F, $CF_3$), – 80,5 (m, 3F, $CF_3$), – 83,3 (m, 2F, $CF_2$—O), – 85,2 (m, 2F, $CF_2$—O), – 113,9 (dd, 1F, —O—CF=$C\underline{F}_2$ trans), – 122,2 (ddm, 1F, —O—CF=$C\underline{F}_2$), – 123,8 (dm, 1F, —CHF—$C\underline{F}_2$, $^2$J = 285 Hz), – 131,4 (ddm, 1F, —CHF—$C\underline{F}_2$—, $^2$J = 285 Hz), – 136,1 (ddm, 1F, —O—CF=$C\underline{F}_2$ cis), – 145,4 (m, 1F, CF), – 213,6 (m, 1F, CHF).

**Ansprüche**

1. Perfluorierte Vinylether mit einem sekundären Wasserstoffatom der Formel I

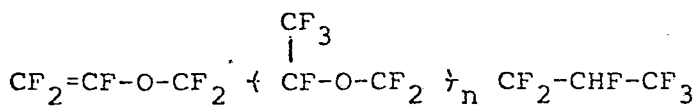 (I)

worin n = 0-5, vorzugsweise 0-3, insbesondere 0-2

2. Polymere mit wiederkehrenden Einheiten der Formel Ia

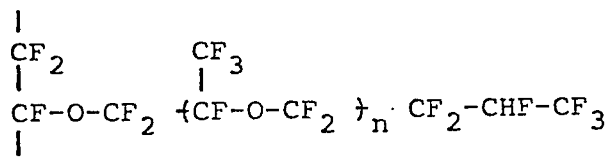 (Ia)

worin n die gleiche Bedeutung wie in Formel I besitzt, ggf. neben anderen bekannten wiederkehrenden Einheiten.

3. Verfahren zur Herstellung der perfluorierten Vinylether mit einem sekundären Wasserstoffatom der Formel I durch

a) Umsetzung von 3-H-Perfluorbutansäurefluorid II

$$FOC—CF_2—CHF—CF_3 \qquad (II)$$

mit Hexafluorpropenepoxid (III)

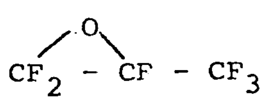 (III)

in Gegenwart mindestens eines ionogenen Fluorids als Katalysator sowie eines inerten aprotisch-polaren Lösungsmittels bei Temperaturen zwischen – 30 und + 100 °C, vorzugsweise zwischen 0 und 50 °C, zu dem Säurefluorid der Formel IV

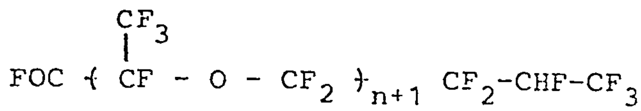 (IV)

worin n die gleiche Bedeutung wie in Formel I besitzt

b) Pyrolyse des Säurefluorids IV als solchem oder nach Umwandlung in das entsprechende Alkalicarboxylat bei Temperaturen zwischen 100 und 600 °C, und

c) Isolierung des bei der Pyrolyse gebildeten Vinylethers der Formel I.

**Claims**

1. Perfluorinated vinyl ethers containing a secondary hydrogen atom of the formula I

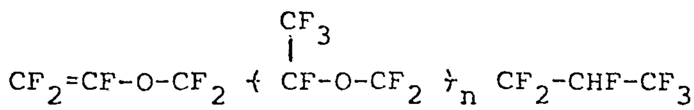 (I)

in which n is 0-5, preferably 0-3 and particularly 0-2.

2. Polymers containing recurring units of the formula Ia

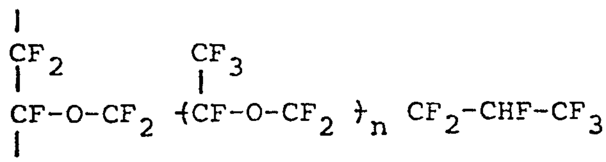 (Ia)

in which n has the same meaning as in formula I, if appropriate together with other known recurring units.

3. A process for the preparation of the perfluorinated vinyl ethers containing a secondary hydrogen atom of the formula I by

a) reacting 3-H-perfluorobutyryl fluoride II

$$FOC—CF_2—CHF—CF_3 \quad (II)$$

with hexafluoropropene epoxide (III)

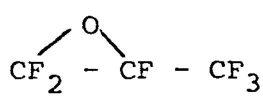 (III)

in the presence of at least one ionic fluoride as catalyst and of an inert, aprotic-polar solvent, at temperatures between − 30 and + 100 °C, preferably between 0 and 50 °C, to give the acid fluoride of the formula IV

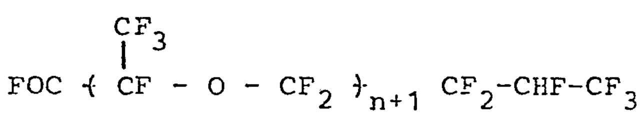 (IV)

in which n has the same meaning as in formula I,

b) pyrolyzing the acid fluoride IV, as such or after conversion into the corresponding alkali metal carboxylate, at temperatures between 100 and 600 °C, and

c) isolating the vinyl ether of the formula I formed in the pyrolysis.

**Revendications**

1. Ether vinylique perfluoré portant un atome d'hydrogène secondaire de formule I

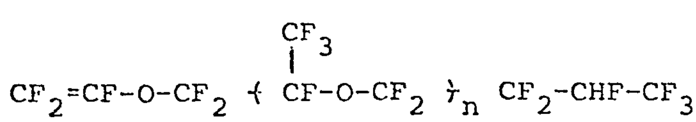 (I)

où n = 0-5, de préférence 0-3, en particulier 0-2.

2. Polymère ayant les motifs répétitifs de formule Ia

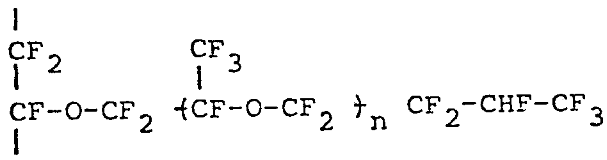

(Ia)

où n a la même signification que dans la formule I, éventuellement outre d'autres motifs répétitifs connus.

3. Procédé pour la préparation des éthers vinyliques perfluorés portant un atome d'hydrogène secondaire de formule I, par

a) Réaction du fluorure de l'acide 3H-perfluorobutyrique II

$$FOC—CF_2—CHF—CF_3 \qquad (II)$$

sur de l'hexafluoro-époxypropène (III)

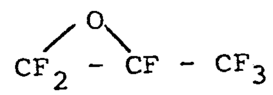

(III)

en présence d'au moins un fluorure ionique en tant que catalyseur, ainsi que d'un solvant aprotique polaire inerte, à des températures comprises entre – 30 et 100 °C, de préférence entre 0 et 50 °C, pour donner le fluorure d'acide de formule IV

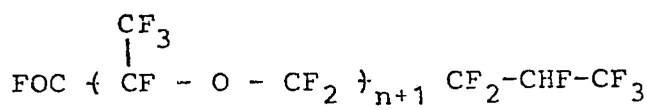

(IV)

où n a la même signification que dans la formule I,

b) pyrolyse du fluorure d'acide IV en tant que tel ou après transformation en le carboxylat de métal alcalin correspondant, à des températures comprises entre 100 et 600 °C, et

c) isolation de l'éther vinylique de formule I formé lors de la pyrolyse.